# EUROPEAN PATENT SPECIFICATION

(11) **EP 2 805 696 B1**
(45) Date of publication and mention of the grant of the patent: **23.11.2016**
(21) Application number: 14169539.5
(22) Date of filing: 22.05.2014
(51) Int. Cl.: A61F 5/01, A43B 3/16

(54) **Stirrup for footwear**
Bügel für Schuhe
Étrier pour chaussures

(30) Priority: 22.05.2013 PT 2013106963
(43) Date of publication of application: 26.11.2014
(73) Proprietor: Amishoes - Calçados LDA, 4800-518 Guimarães (PT)
(72) Inventor: Costa Teixeira, Luís Miguel, 4805-184 Guimarães (PT); Garcia de Valadares Souto, António Pedro, 4820-360 Fafe (PT); Flores Fernandes, João Paulo, 4710-103 Braga (PT); Mendes Ferrão, Renato Manuel, 4810-242 Guimarães (PT); Gomes Peixoto, Joaquim Jorge, 4700-038 Braga (PT); Novo de Moura, Bruno Emanuel, 4700-383 Braga (PT)
(74) Representative: Silvestre de Almeida Ferreira, Luís Humberto

(56) References cited:
- AU-A- 5 068 073
- US-A- 3 732 861
- US-A- 3 805 773
- US-A1- 2006 009 725

## Description

### Technical Field

The present description refers to a mechanical device for orthopedic footware. More specifically it consists of a stirrup which allows the connection between orthopedic footware and the orthoses of users with motor disability.

### Background Art

The stirrup is the orthoses connecting element to footware. Orthoses can be small (in relation to the ankle), medium-size (ankle and knee), or large (ankle, knee and hip). Generally, the stirrup is usually fastened to the sole, in the area under the heel not being able to be removed once it has been placed into the sole.

Traditional stirrups are, mostly, made of stainless steel, what makes them very heavy. Usually, they are provided in a planar format and subsequently bent to the needs of each patient. They usually have a structure of solid steel which is directly fastened to the shoe sole, in the anterior section of the heel, being able to contour the heel of the user and can have an additional device aiming at fastening it to footware.

Regarding the sizes of this component, there are in the market standardised ranges of sizes (standard), being thus able to respond to the different needs of each individual. Besides these measurements, there are different types of stirrups with distinct shapes at their extreme ends. Along with different articulations, the stirrup can solely promote dorsiflexion and/or plantar flexion only. There are also articulations which allow both of the above mentioned movements (i.e. double action) and still the complete restriction of the foot to any type of movement.

Many stirrups have different functioning critical points, not being thus able to provide a positive answer to the above mentioned factors. The stirrup can many times undergo a material rupture in a short time span (depending on the user and on its use), in areas of high concentration of stress. This situation becomes one of the priority targets of this disclosure, since when this happens the user can suffer accidents and/or restraint and is prevented from performing his/her daily activities.

Furthermore, conventional stirrups have the following characteristics:
- The component is too heavy, which causes a tremendous discomfort and an increased energy expenditure by the user;
- The aesthetics has been implement for many years, and lacks innovation;
- High costs due to the fact that part of the manufacturing process is handcrafted depending on the needs of each user.

Although the stirrup systems that have already been developed and patented have helped the users, one finds that there are a number of limitations. As already mentioned, stirrup ruptures happen in the area where they are bent in an angle of about 90°, and this rupture is mainly due to the formation of fragile areas, because of the bending when adjusting the stirrup to the needs of the patient. Conventional stirrups have a high mass, since they are made of steel with a high density and are usually fastened to footware using spikes and/or rivets, what causes the mechanism to be of high removing difficulty by the user.

US 3,805,773 discloses a training assist brace including a conventional calliper stirrup.

### Summary

The present disclosure consists of a mechanical device, referred to as stirrup, for use in tailor-made orthopedic footware, which is in turn used in orthoses for the inferior members, which can be: small (in relation to the ankle), medium-size (ankle and knee), or large (ankle, knee and hip).

This stirrup can be developed so as to be used in almost all situations, that is: it can be length and height graduated, to reach all types of feet (length, height of pronation or supination angles). It can also be manufactured, according to medical prescription, when the movement limitation, that is, it can be used in patients, whose indication is the inexistence of ankle movement, in patients with ankle movement limitation or in patients with free movement. This characteristic is achieved when alternating the geometrical shape of the extreme end of the stirrup. With this disclosure, on the one side, a reduction of about 50% by weight in relation to conventional stirrups (usually made of steel) has been achieved, and, on the other side, the presented geometry has eliminated the points of higher fragility, thus making the device safer and having a longer lifespan. The fastening of the stirrup to the boot is done using a T-shaped aluminium structure, where two screws perpendicular to each other are placed into the boot - a screw of a lateral fastening opening to the sole to the other lateral fastening opening to the sole, and a screw in the posterior fastening opening to the sole. Three screws are also used to fasten the stirrup, which are placed on a support insole which has a metallic plate for limitation of the lateral movement of the stirrup.

The present disclosure consists of a stirrup to be adapted to patients with musculoskeletal disabilities, such as, for instance, poliomyelitis and multiple sclerosis. Since the stirrup is a component which when associated to other elements has as main goal the walking correction of the user, improving his/her performance in terms of comfort and well-being, as well as enabling daily activities to be carried out in a more normal way, the development of equipment capable of meeting all these situations becomes indispensable.

The present disclosure consists of a stirrup which can be used in orthoses which can be small (ankle), medium-size (ankle and knee), or large (ankle, knee and hip), so that the shortcomings of the above mentioned systems are eliminated.

A stirrup for footware, for integration in the shoe itself and that comprises a single piece composed of a U-shaped semi-circular inferior part, configured to contour the heel, wherein each of the extreme ends comprises a perpendicular arm; and a T-shaped planar piece, placed in the inferior part of the semi-circular piece are described.

In an embodiment of the stirrup of the present disclosure, the T-shaped piece can be connected to the U-shaped semi-circular part through removable screws, clamps, grooves, rivets, gluing, welding or combinations thereof.

In an embodiment of the stirrup of the present disclosure, the T-shaped piece can comprise openings (34) for insertion of removable screws for connection to the insole, in particular the openings being three.

In an embodiment of the stirrup of the present disclosure, the T-shaped piece can be 2 millimetres thick.

In an embodiment of the stirrup of the present disclosure, it can comprise one or more openings in the inferior part of the arms, in the U-shaped area, for integration of removable screws to fasten the stirrup to the sole.

In an embodiment of the stirrup of the present disclosure, it can comprise one or more openings in the superior part of the arms for integration of an orthosis.

In an embodiment of the stirrup of the present disclosure, the U-shaped semi-circular part can be 3 millimetres thick.

In an embodiment of the stirrup of the present disclosure, the stirrup can be in aluminium, in particular in aluminium 7075-T6 (SN).

In an embodiment of the stirrup of the present disclosure, each of the perpendicular arms of the two extreme ends of the U-shaped piece can be configured to vertically extend along the inner and outer part of the leg.

In an embodiment of the stirrup of the present disclosure, the T-shaped piece can be configured to extend between the lateral ends of the footware article.

In an embodiment of the stirrup of the present disclosure, the U-shaped piece can be a plate configured to contour the heel along one of the plate faces.

In an embodiment of the stirrup of the present disclosure, the T-shaped piece can be configured in an inverted V angle for allowing a predefined articulation angle of the stirrup.

In an embodiment of the stirrup of the present disclosure, the piece wherein the stirrup can configured to be placed into the sole of a footware article.

A footware article which comprises the described stirrup is also described, wherein the sole of the footware article is hewn in a thickness substantially equal to the thickness of the stirrup such that the stirrup is placed into the sole of the footware article.

The stirrup that has been developed is characterised by the fact that the selected material can be aluminium, since it is a material that is more resistant to stress. Aluminium 7075-T6 (SN) should be preferably selected, whose main characteristics are: stress rupture 5.7e+008 N/m2, modulus of elasticity 7.2e+010 N/m2, Poisson's ratio 0.33 density 2810 kg/m3, shear stress 2.69e+010 N/m2 coefficient of thermal expansion 2.36e-005/Kelvin.

Geometrically, the stirrup can be composed of a single aluminium piece (through bending), wherein the inferior part is semi-circular (U-shaped) (26), each extreme end having one perpendicular arm (9) for connection to the orthoses. One therefore finds that there is not any 90° angle through bending and, as a consequence, the stress that occurs will not take place in that critical point of the ordinary stirrups (which leads to the non-existence of device rupture). As previously mentioned, ruptures in conventional stirrups are a result of solicitations caused by body weight and ankle pronation in a cyclic movement. The continuous application of these strains leads to a significant material wear till its rupture, and it will cease to exist.

The stirrup contours the heel of the user, and the arms will allow the connection to the orthosis for the correct walking of the user. There is also a T-shaped piece that is linked to the inferior part of the stirrup, more precisely, linked to the U-shaped area, in order to allow the correct placing of the foot on the ground. This T-shaped auxiliary piece also allows fastening and it prevents the lateral movement of the stirrup while walking. While using this very same mechanism (T-shaped), the stirrup will also be fastened to the sole of footware, both transversally and longitudinally, through removable screws. The use of removable screws, both when fastening the stirrup to the sole of the footware, and in the T fastening device in the insole, allows the user to be able to remove the stirrup and place it into other footware in an easier and faster way.

The stirrup can be placed into the sole of footware, so that it is always hidden, what makes the design of the article look better, and the sole thickness has to be equivalently hewn to the thickness of the stirrup (3 mm) and has to have a minimum height of 20 mm.

As a procedure, the stirrup can be initially fastened to an insole which can be of any type of material as, for instance, be composed of recycled leather.

The articulation of the stirrup can be developed to allow different movements by the user, namely:
- Free movement: extreme end of the stirrup has to be made in inverted V,
- With limited movement: according to medical prescription, the orthopedician can hew the extreme end so as to reach the prescribed angle and, thus, achieve the desired dorsiflexion movement.
- No movement: the extreme end of the stirrup has to have two right angles and reach a 90° angle with the lateral arm.

The stirrup will be fastened to the orthosis through removable screws.

The present disclosure provides the user with a set of advantages, since the user himself will be able to fasten it in an easy and fast way. The device can also be quickly connected to small, medium-size or large orthoses, it is a system whose mass is relatively low. It is also possible to use it in patients with significant dysmetria (for instance, high differences between the height of the lower limbs) or in patients who do not suffer from dysmetria, thus being able to be used in conventional footware.

The disclosed embodiments are combinable. The following claims set out particular embodiments of the disclosure.

### Brief Description of the Drawings

For illustrating the invention and for showing its advantages, below is a brief description of the drawings which should not be seen as limiting the scope of the disclosure.

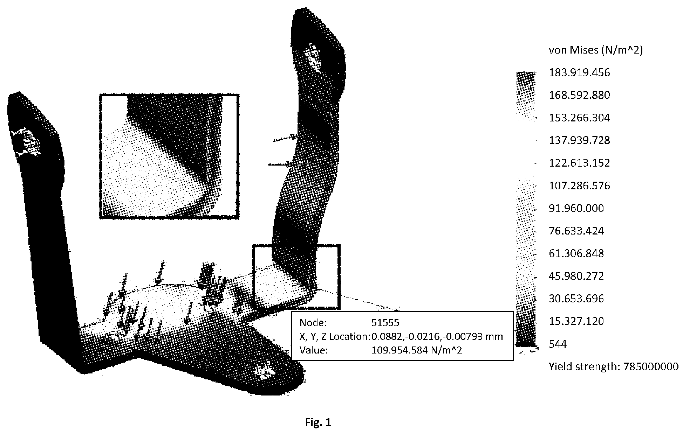

Fig. 1 (representation of the stress of a conventional stirrup) shows the yield points which occur in a conventional stirrup, the area of higher stress being the one which is in an area where there is an angle of almost 90 degrees - the value in this area is of 100 MPa.

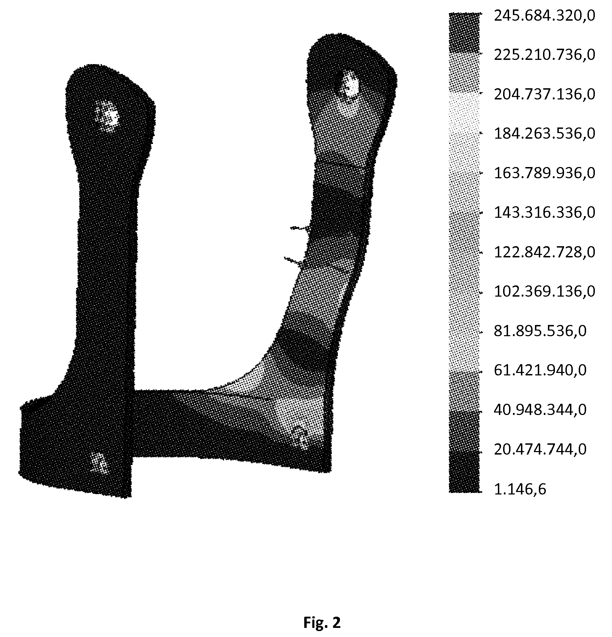

Fig. 2 shows the yield points which occur in the object of this disclosure, the area of higher stress is the one in the fastening holes in the sole and the value is of approximately 228 MPa, whereas in the previous figure the highest yield point is in the structure itself. When the highest yield point is located in the fastening hole, it does not cause any harm to the device nor to its user.

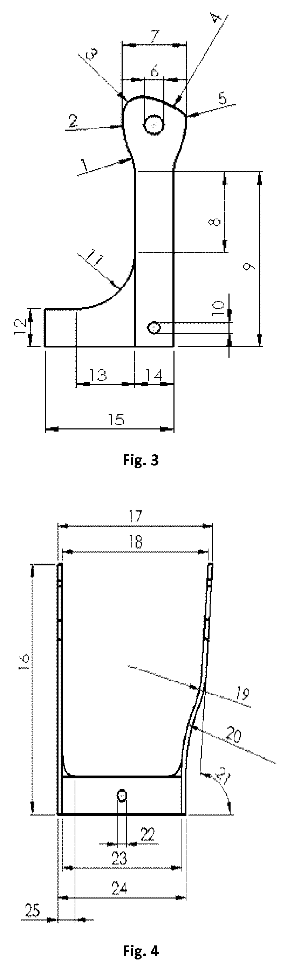

Fig. 3 (right lateral front) shows the right lateral front, wherein (1) corresponds to the bend radius; (2) bend radius; (3) bend radius; (4) bend radius; (5) bend radius; (6) diameter of the hole for orthosis fitting; (7) articulation width; (8) segment length; (9) length of the base of the articulation; (10) diameter of the hole for sole fitting; (11) bend radius; (12) height of the inferior part of the stirrup; (13) width of the segment; (14) width of the arm, and (15) width of the stirrup.
Fig. 4 (main front) shows the main front, wherein (16) corresponds to the overall height of the left side; (17) to the exterior width of the stirrup; (18) to the inner width; (19) external bend radius; (20) internal bend radius; (21) to the distance from the base to the curvature; (22) diameter of the fastening hole to the sole; (23) to the internal width; (24) to the external width; and (25) to the distance from the outside to the curvature limit.

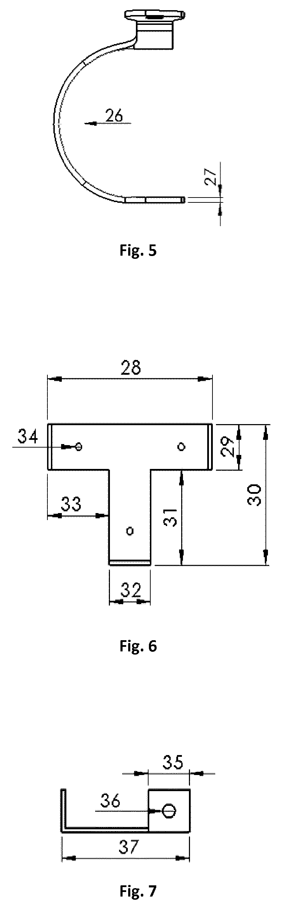

Fig. 5 (upper front) shows the upper front, wherein (26) corresponds to the bend radius and (27) to the thickness.
Fig. 6 (upper front) shows the upper front of the T piece, wherein (28) corresponds to the outer width of the support T device; (29) outer width of the tab of the support T device; (30) overall outer length; (31) transverse tab length; (32) outer width of the tab of the T support device; (33) tab lengths; (34) diameter of the fastening openings to the insole.
Fig. 7 (lateral view) shows the lateral view of the T piece, wherein (35) corresponds to the tab width; (36) to the diameter of the fastening side opening to the sole and (37) T device overall length.

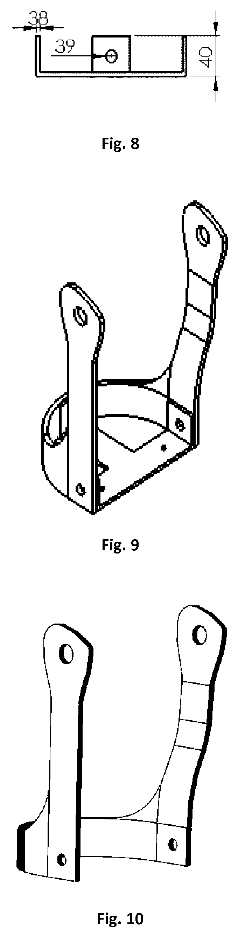

Fig 8 (main front) shows the main front of the T piece, wherein (38) corresponds to the T device thickness; (39) diameter of the posterior fastening opening to the sole and (40) tab height.
Fig. 9 (3D drawing of the stirrup and corresponding T-shaped fastening system) represents the 3D drawing of the stirrup and T-shaped device set.
Fig. 10 (3D drawing of the stirrup) corresponds to the 3D drawing of the stirrup only.

### Detailed Description

The following example should not be seen as limiting the scope of the disclosure.

The new stirrup geometry that has been presented is capable of reducing about 50% of the mass, comparing to the conventional models. This reduction is due to the use of an aluminium alloy, with a much lower density and a yield stress equivalent to the material used (AISI 420 stainless steel). As it is less heavy, it allows a more natural motion of the user. The removal of the support base of the conventional stirrups allows a great reduction of the shear stress which exists in the bending area of the fitting of the base to the lateral tab.

This stirrup allows a fast and easy manufacture and assembly, and one single small bent plate is needed to sustain the fastening elements.

The following dimensions are needed to develop the functional stirrup:

| **Number** | **Description** | **Value (mm)** |
|---|---|---|
| 1 | Bend radius | 30.16 |
| 2 | Bend radius | 47.61 |
| 3 | Bend radius | 10.00 |
| 4 | Bend radius | 42.49 |
| 5 | Bend radius | 4.00 |
| 6 | Diameter of the hole for orthosis fitting | 10.00 |
| 7 | Articulation width | 32.67 |
| 8 | Segment length | variable, in particular 35.00-60.00 preferably 43.00 |
| 9 | Articulation base length | variable, in particular 85.00-110.00 preferably 93.00 |
| 10 | Diameter of the hole for sole fitting | 6.00 |
| 11 | Bend radius | 30.00 |
| 12 | Height of the inferior part of the stirrup | 20.00 |
| 13 | Segment width | 20 |
| 14 | Arm width | 30 |
| 15 | Stirrup width | variable, in particular 66.10 |
| 16 | Left side overall height | variable, in particular 125.00-150.00, preferably 132.68 |
| 17 | Stirrup outer width | variable |
| 18 | Inner width | variable |
| 19 | External bend radius | variable |
| 20 | Internal bend radius | variable |
| 21 | Distance from the base to the curvature | 85.67 |
| 22 | Diameter of the fastening hole to the sole | 6.00 |
| 23 | Internal width | variable |
| 24 | External width | variable |
| 25 | Distance from the outside to the curvature limit | 11.59 |
| 26 | Bend radius | variable |
| 27 | Thickness | 3.00 |
| 28 | Outer width of the T support device | variable |
| 29 | Outer width of the tab of the T support device | 20.00 |
| 30 | Overall outer length | variable |
| 31 | Transverse tab length | variable |
| 32 | Outer width of the tab of the T support device | 20.00 |
| 33 | Tab lengths | variable |
| 34 | Diameter of the fastening openings to the insole | 3.00 |
| 35 | Tab width | 20.00 |
| 36 | Diameter of the fastening side opening to the sole | 6.00 |
| 37 | T device overall length | variable |
| 38 | T device thickness | 2.00 |
| 39 | Diameter of the posterior fastening opening to the sole | 6.00 |
| 40 | Tab height | 20.00 |

## Claims

1. Stirrup for shoes or footwear article, for integration in the shoe or footwear article itself, comprising:
- a single piece composed of a U-shaped semi-circular inferior part, configured to contour the heel, wherein each of its extreme ends comprises a perpendicular arm; and
- a T-shaped planar piece, placed in the inferior part of the semi-circular piece; wherein each of the perpendicular arms of the two extreme ends of the U-shaped piece are configured to vertically extend along the inner and outer part of the leg.

2. Stirrup according to the previous claim wherein the T-shaped piece is connected to the U-shaped semi-circular part through removable screws, clamps, grooves, rivets, gluing, welding or combinations thereof.

3. Stirrup according to any of the previous claims wherein the T-shaped piece comprises openings (34) for insertion of removable screws for connection to the insole, in particular the openings being three.

4. Stirrup according to any of the previous claims wherein the T-shaped piece is 2 millimetres thick.

5. Stirrup according to any of the previous claims that comprises one or more openings in the inferior part of the arms, in the U-shaped area, for integration of removable screws to fasten the stirrup to the sole.

6. Stirrup according to any of the previous claims that comprises one or more openings in the superior part of the arms for integration of an orthosis.

7. Stirrup according to any of the previous claims wherein the U-shaped semi-circular part is 3 millimetres thick.

8. Stirrup according to any of the previous claims wherein the stirrup is in aluminium.

9. Stirrup according to the previous claim **characterised in that** the stirrup is in aluminium 7075-T6 (SN).

10. Stirrup according to any of the previous claims wherein the T-shaped piece is configured to extend between the lateral ends of the footwear article.

11. Stirrup according to any of the previous claims wherein the U-shaped piece is a plate configured to contour the heel along one of the plate faces.

12. Stirrup according to any of the previous claims wherein the T-shaped piece is configured in an inverted V angle for allowing a predefined articulation angle of the stirrup.

13. Stirrup according to any of the previous claims configured to be placed into a sole of a footwear article.

14. Footwear article comprising the stirrup according to the previous claim, wherein the sole of the footwear article is hewn in a thickness substantially equal to the thickness of the stirrup such that the stirrup is placed into the sole of the footwear article.

## Patentansprüche

1. Steigbügel für Schuhe oder Schuherzeugnisse, für die Integration in den Schuh oder Schuherzeugnisse selbst, enthaltend:
- ein Einzelstück bestehend aus einem U-förmigen halbrunden unteren Teil, gestaltet für die Fersenform, worin jedes Ende einen senkrechten Arm umfasst;
- ein T-förmiges flaches Stück, welches auf den unteren Teil des halbrunden Stücks aufgelegt ist;
worin jeder der senkrechten Arme der zwei Enden des U-förmigen Teils so angeordnet ist, um vertikal längs des inneren und äußeren Teil des Beines zu verlaufen.

2. Steigbügel nach dem vorangegangenen Anspruch, worin das T-förmige Teil an den U-förmigen halbrunden Teil durch abnehmbare Schrauben, Klemmen, Rillen, Nieten, Kleben, Schweißen oder in Kombinationen davon verbunden ist.

3. Steigbügel nach irgendeinem der vorangegangenen Ansprüche, worin das T-förmige Teil Ösen (34) für das Einfügen von abnehmbaren Schrauben für die Verbindung zur Einlegesohle enhält, im Besonderen die drei Öffnungen.

4. Steigbügel nach irgendeinem der vorangegangenen Ansprüche, worin das T-förmige Teil 2 Millimeter dick ist.

5. Steigbügel nach irgendeinem der vorangegangenen Ansprüche, welcher eine oder mehrere Ösen am unteren Teil der Arme enthält, im U-förmigen Bereich, für die Integration abnehmbarer Schrauben zur Befestigung des Steigbügels an der Sohle.

6. Steigbügel nach irgendeinem der vorangegangenen Ansprüche, welcher eine oder mehrere Ösen im oberen Teil für die Integration einer Orthese enthält.

7. Steigbügel nach irgendeinem der vorangegangenen Ansprüche, worin ein U-förmiger halbrunder Teil 3 Millimeter dick ist.

8. Steigbügel nach irgendeinem der vorangegangenen Ansprüche, worin der Steigbügel aus Aluminium ist.

9. Steigbügel nach dem vorangegangenen Anspruch, welcher dahingehend beschrieben ist, dass der Steigbügel aus Aluminium 7075-T6 (SN) ist.

10. Steigbügel nach irgendeinem der vorangegangenen Ansprüche, worin das T-förmige Stück so angeordnet ist, dass es zwischen den Seitenenden des Schuherzeugnisses verläuft.

11. Steigbügel nach irgendeinem der vorangegangenen Ansprüche, worin das U-förmige Stück eine Trittplatte für die Fersenform entlang einer der Plattenoberflächen ist.

12. Steigbügel nach irgendeinem der vorangegangenen Ansprüche, worin das T-förmige Stück einen umgekehrten V-Winkel darstellt, damit ein vorgegebener Beugungswinkel des Steigbügels möglich ist.

13. Steigbügel nach irgendeinem der vorangegangenen Ansprüche, eingerichtet um unter der Sohle eines Schuherzeugnisses angebracht zu werden.

14. Schuherzeugnis, enthält einen Steigbügel nach dem vorangegangenen Anspruch, worin die Sohle eines Schuherzeugnisses so dick wie substantiell die Dicke des Steigbügels beschaffen ist, dahingehend, dass der Steigbügel in die Sohle des Schuherzeugnisses gelegt wird.

## Revendications

1. Sangle pour tous types de chaussures, pour une intégration à la propre chaussure, comprenant:
- une pièce unique composée d'une partie inférieure semi-circulaire en forme de « U », configurée pour contourner le talon, dans laquelle chacune des extrémités comprend un bras perpendiculaire;
et
- une pièce plane en forme de « T », placée sur la partie inférieure de la pièce semi-circulaire;
dans laquelle chacun des bras perpendiculaires des deux extrémités de la pièce en forme de « U » sont configurées de façon à s'étendre verticalement le long de la partie intérieure et extérieure de la jambe.

2. Sangle selon la revendication précédente dans laquelle la pièce en forme de « T » est reliée à la partie semi-circulaire en forme de « U » par des vis amovibles, attaches, encoches, rivets, par encollage, soudage, ou par combinaison de ceux-ci.

3. Sangle selon l'une quelconque des revendications précédentes dans laquelle la pièce en forme de « T » comprend des ouvertures (34) pour l'insertion de vis amovibles pour un rattachement à la semelle intérieure, les ouvertures étant précisemment au nombre de trois.

4. Sangle selon l'une quelconque des revendications précédentes dans laquelle la pièce en forme de « T » a une épaisseur de 2 millimètres.

5. Sangle selon l'une quelconque des revendications précédentes qui comprend une ou plusieurs ouvertures dans la partie inférieure des bras, dans la zone en forme de « U », pour l'intégration de vis amovibles afin d'attacher la sangle à la semelle.

6. Sangle selon l'une quelconque des revendications précédentes qui comprend une ou plusieurs ouvertures dans la partie supérieure des bras pour l'intégration dune orthèse.

7. Sangle selon l'une quelconque des revendications précédentes dans laquelle la pièce semi-circulaire en forme de « U » a une épaisseur de 3 millimètres.

8. Sangle selon l'une quelconque des revendications précédentes dans laquelle la sangle est en aluminium.

9. Sangle selon la revendication précédente **caractérisée par le fait que** la sangle soit en aluminium 7075-T6 (SN).

10. Sangle selon l'une quelconque des revendications précédentes dans laquelle la pièce en forme de « T » est configurée pour s'étendre entre les extrémités latérales de la chaussure.

11. Sangle selon l'une quelconque des revendications précédentes dans laquelle la pièce en forme de « U » est une plaque configurée pour contourner le talon le long de l'une des faces de la plaque.

12. Sangle selon l'une quelconque des revendications précédentes dans laquelle la pièce en forme de « T » est configurée dans un angle de V inversé afin de permettre un angle d'articulation de la sangle prédéfini.

13. Sangle selon l'une quelconque des revendications précédentes configurée pour être placée dans la semelle d'une chaussure.

14. Chaussure comprenant la sangle selon la revendication précédente, dans laquelle la semelle de la chaussure est taillée dans une épaisseur substanciellement égale à l'épaisseur de la sangle, de façon à ce que la sangle soit placée dans la semelle de la chaussure.
